# EUROPEAN PATENT APPLICATION

(11) **EP 4 768 491 A1**
(43) Date of publication of application: **01.07.2026**
(21) Application number: 24855875.1
(22) Date of filing: 22.08.2024
(51) Int. Cl.: C07H 15/04, C07H 1/06, A61K 31/7028, A61P 29/00, A61P 11/00, A61P 13/12, A61P 1/16, A61P 1/18, A61P 9/10, A61P 7/02, A61P 17/00, A61P 17/06, A61P 9/00, A61P 1/00, A61P 19/02

(54) **CRYSTALLINE FORM OF COMPOUND, PREPARATION THEREOF, COMPOSITION COMPRISING SAME, AND USE THEREOF**

(30) Priority: 23.08.2023 CN 202311069099; 23.08.2023 CN 202311065293
(71) Applicant: Grand Pharma (China) Co., Ltd., Wuhan, Hubei 430034 (CN); Wuhan Wuyao Sci & Tech Co., Ltd, Wuhan, Hubei 430074 (CN)
(72) Inventor: SHU, Min, Wuhan, Hubei 430034 (CN); RAO, Xiang, Wuhan, Hubei 430034 (CN); DONG, Meng, Wuhan, Hubei 430034 (CN); ZHANG, Xiao, Wuhan, Hubei 430034 (CN); LIAO, Hang, Wuhan, Hubei 430034 (CN); LIU, Xianbo, Wuhan, Hubei 430034 (CN)
(74) Representative: Johnson, Stephen William
(86) International application number: PCT/CN2024/113973
(87) International publication number: WO 2025/040154

(57) **Abstract**

Disclosed in the present invention are a crystalline form of a compound, a preparation method therefor, an active pharmaceutical ingredient or composition comprising same, and a use thereof in the preparation of an extracellular histone inhibitor. The crystalline form of the compound of the present application has high crystallinity, good solid properties and high stability, crystallization conditions are mild, the operation is simple, the process is stable, the cost is low, and the crystalline form of the compound is adapted to be stored as an active pharmaceutical ingredient and produce a formulation product.

## Description

### TECHNICAL FIELD

The present invention relates to the field of medicine, and specifically to a crystalline form of compound, preparation thereof, composition comprising same and use thereof.

### BACKGROUND ART

Sepsis is a common critical illness in ICU with high mortality rate. Globally, there is no recognized effective therapeutic drug for sepsis, and the clinic is mainly focused on symptomatic supportive treatment. Sepsis is a medical challenge that needs to be solved urgently in the clinic. Patent NO. CN201880080079.X discloses that a polyanionic cellobioside sulfate compound shows good therapeutic effects for a variety of diseases including sepsis, and has entered the clinical research stage, showing good development value.

An amorphous form of this compound can be obtained according to the methods reported in the literature (Probst, Katrin C. SYNTHESIS AND CONFORMATIONAL INVESTIGATIONS OF SULFATED CARBOHYDRATES[1][J].Cheminform, 2001, 20(7-8):549-560.) as well as Patent NO. CN201880080079.X and Patent Application NO. WO2019113646A1, but it has the disadvantages of low purity, poor stability, poor fluidity, etc., and there is no report of study on its crystalline form.

### SUMMARY OF THE INVENTION

In view of the aforementioned problems existing in the prior art, the present invention provides a technical solution to the above problems.

A first aspect of the present invention provides a crystalline form of sodium β-O-methyl-D-cellobioside heptasulfate, which is selected from one of crystalline form A and crystalline form C, or a mixture thereof; wherein,
the crystalline form A is characterized by a Cu-Kα radiation X-ray powder diffraction pattern with characteristic diffraction peaks present at 20 angles comprising 19.60±0.2°, 22.64±0.2°, and 25.45±0.2°; and
the crystalline form C is characterized by a Cu-Kα radiation X-ray powder diffraction pattern with characteristic diffraction peaks present at 20 angles comprising 20.80±0.2°, 23.38±0.2°, and 23.64±0.2°.

A second aspect of the present invention provides an active pharmaceutical ingredient comprising the crystalline form of the present application as described above.

A third aspect of the present invention provides a composition comprising the crystalline form of the present application as described above and one or more pharmaceutically acceptable excipients.

In a fourth aspect of the present invention, there is provided use of the crystalline form, the active pharmaceutical ingredient, or the composition of the present application in the preparation of an extracellular histone inhibitor.

In a fifth aspect of the present invention, there is provided use of the crystalline form, the active pharmaceutical ingredient, or the composition of the present application in the preparation of a medicament for treating or preventing an extracellular histone-mediated disease or condition.

In a sixth aspect of the present invention, there is provided use of the crystalline form, the active pharmaceutical ingredient, or the composition as described above for treating a histone-mediated disease.

In a seventh aspect of the present invention, there is provided use of the crystalline form, the active pharmaceutical ingredient, or the composition of the present application in the preparation of a medicament for treating or preventing a following disease, the disease being selected from the group consisting of: inflammation, severe pneumonia, sepsis, systemic inflammatory response syndrome, acute pancreatitis, acute kidney injury, acute lung injury, acute liver injury, acute respiratory distress syndrome, ischemia-reperfusion injury, atherosclerosis, deep vein thrombosis, ischemic injury, ischemic stroke, multiple sclerosis, systemic lupus erythematosus, ankylosing spondylitis, psoriatic arthritis, ulcerative colitis, Crohn's disease and rheumatoid arthritis.

In an eighth aspect of the present invention, there is provided a method of treating a disease, characterized by administering to a patient a therapeutically effective amount of the crystalline form, the active pharmaceutical ingredient, or the composition. The disease is selected from the group consisting of: inflammation, severe pneumonia, sepsis, systemic inflammatory response syndrome, acute pancreatitis, acute kidney injury, acute lung injury, acute liver injury, acute respiratory distress syndrome, ischemia-reperfusion injury, atherosclerosis, deep vein thrombosis, ischemic injury, ischemic stroke, multiple sclerosis, systemic lupus erythematosus, ankylosing spondylitis, psoriatic arthritis, ulcerative colitis, Crohn's disease and rheumatoid arthritis.

A ninth aspect of the present invention provides a method for purifying the compound, sodium β-O-methyl-D-cellobioside heptasulfate, through any of the following methods:
(1) Anti-solvent addition purification method, specifically: a first solvent is a good solvent and a second solvent is an anti-solvent, the crude product of the compound, sodium β-O-methyl-D-cellobioside heptasulfate, is purified by using the anti-solvent addition method, to afford a purified compound;
(2) Cooling crystallization purification method, specifically: the compound, sodium β-O-methyl-D-cellobioside heptasulfate, is added into a mixed solvent Y and dissolved at high temperature, followed by cooling crystallization to afford a purified compound; the mixed solvent Y is a mixture of the first solvent and the second solvent;
(3) Slurrying purification method, specifically: the crude product of the compound, sodium β-O-methyl-D-cellobioside heptasulfate, is purified by slurrying purification using a mixed solvent X as the solvent, to afford a purified compound, the mixed solvent X is a mixture of the first solvent and the second solvent;
wherein the first solvent is water, and the second solvent is selected from one or more of C1 to C6 alcohols, acetone and acetonitrile.

In a tenth aspect of the present invention, there is provided a method for preparing the crystalline form C as described above, comprising the steps of adding the compound, sodium β-O-methyl-D-cellobioside heptasulfate, to a solvent Z and stirring, isolating the solids to obtain the crystalline form C; wherein the solvent Z is a mixture of a first solvent and a second solvent; and the first solvent is water and the second solvent is 1,4-dioxane.

The advantageous effects of the present invention are as follows:
(1) The three crystalline forms A to C were screened out after nearly 100 conditions, indicating that the crystalline form A and the crystalline form C of the present application are not readily available *per se.*
(2) In the actual production process, the amorphous form requires low-temperature storage and transportation. In contrast, the crystalline form A and the crystalline form C exhibit stability advantages under high temperature and light conditions, which allows for the relaxation of storage and transportation temperature restrictions for the active pharmaceutical ingredient (it can be stored at room temperature). This offers greater convenience, cost-effectiveness, and energy savings.
(3) In comparison to the drawbacks associated with the preparation of the crystalline form B, which requires the use of N,N-dimethylformamide, suffers from severe solvent residue that is difficult to remove, and is not amenable to industrial scale-up, the crystallization agents used for the crystalline form A and the crystalline form C are common, readily available on the market, and low-cost, moreover, the crystallization conditions are mild, the operation is simple, the process is robust, and the overall cost is low, making them suitable for industrial scale-up and having high commercial viability.
(4) In comparison to the amorphous form and the crystalline form B, the crystalline form A of the present application exhibits good solid-state properties, resistance to crystal transformation, and is thermodynamically stable. Furthermore, the crystalline form A exhibits good flowability, offering advantages as an active pharmaceutical ingredient for formulation preparation. The crystalline form A can be directly compressed for tablet manufacturing, with satisfactory tableting performance showing no problems such as loose or broken tablets. The tablet weight variation meets requirements, and the tablets exhibit good dissolution behavior.
(5) In comparison to the amorphous form and the crystalline form B, which exhibits problems such as poor tablet friability, susceptibility to cracking, unstable tablet weight variation, slow dissolution rate, and low dissolution during the dry granulation compression process, the crystalline form C demonstrates better tableting performance, with advantages in both dissolution extent and dissolution rate, making it more suitable for formulation preparation.
(6) During the scale-up of compound purification, since the compound has high solubility in water, polymer-induced crystallization sometimes exhibits problems such as a failure to crystallize, resulting in an unstable process. By employing the specific antisolvent type of the present application, the compound can be effectively purified through antisolvent addition crystallization, cooling crystallization, or slurrying purification. The process achieves a purity of 98% or more with mild conditions, simple operation, stable procedure, and high feasibility for commercialization. In specific embodiments, the purification can directly afford the crystalline form A as an active pharmaceutical ingredient for storage and formulation preparation.

### BRIEF DESCRIPTION OF THE DRAWINGS

Fig. 1 shows the H¹ NMR spectrum of the compound prepared in Example 1.
Fig. 2 shows the X-ray powder diffraction pattern (XRPD) of the compound prepared in Example 1.
Fig. 3 shows the X-ray powder diffraction pattern (XRPD) of the crystalline form A.
Fig. 4 shows the thermogravimetric analysis (TGA-DTG) curve of the crystalline form A.
Fig. 5 shows the differential scanning calorimetry (DSC) curve of the crystalline form A.
Fig. 6 shows the H¹NMR spectrum of the crystalline form A.
Fig. 7 shows the X-ray powder diffraction pattern (XRPD) of the crystalline form B.
Fig. 8 shows the thermogravimetric analysis (TGA-DTG) curve of the crystalline form B.
Fig. 9 shows the differential scanning calorimetry (DSC) curve of the crystalline form B.
Fig. 10 shows the H¹NMR spectrum of the crystalline form B.
Fig. 11 shows the morphology of the crystalline form A.
Fig. 12 shows the morphology of the crystalline form B.
Fig. 13 shows the H¹NMR spectrum of the crystalline form C.
Fig. 14 shows the X-ray powder diffraction pattern (XRPD) of the crystalline form C.
Fig. 15 shows the thermogravimetric analysis (TGA-DTG) curve of the crystalline form C.
Fig. 16 shows the differential scanning calorimetry (DSC) curve of the crystalline form C.

### DETAILED DESCRIPTION OF THE INVENTION

In a specific embodiment, the crystalline form A is characterized by a Cu-Kα radiation X-ray powder diffraction pattern with characteristic diffraction peaks present at 20 angles further comprising any one or more of 20.69±0.2°, 22.32±0.2°, 25.74±0.2°, 26.99±0.2°, and 29.70±0.2°.

In a specific embodiment, the crystalline form A is characterized by a Cu-Kα radiation X-ray powder diffraction pattern with characteristic diffraction peaks present at 20 angles further comprising any one or more of 10.40±0.2°, 11.38±0.2°, 14.98±0.2°, 17.97±0.2°, 20.33±0.2°, 23.51±0.2°, 24.61±0.2°, 28.75±0.2°, 34.00±0.2°, and 35.63±0.2°.

In a specific embodiment, the crystalline form A is characterized by a Cu-Kα radiation X-ray powder diffraction pattern with characteristic diffraction peaks present at 20 angles further comprising any one or more of 7.63±0.2°, 2.08±0.2°, 13.45±0.2°, 14.12±0.2°, 16.27±0.2°, 16.75±0.2°, 17.11±0.2°, 27.58±0.2°, 28.04±0.2°, 29.22±0.2°, 31.18±0.2°, 31.80±0.2°, 32.62±0.2°, 33.05±0.2°, 33.41±0.2°, 35.03±0.2°, 37.12±0.2°, 38.04±0.2°, 38.40±0.2°, 38.77±0.2°, and 39.33±0.2°.

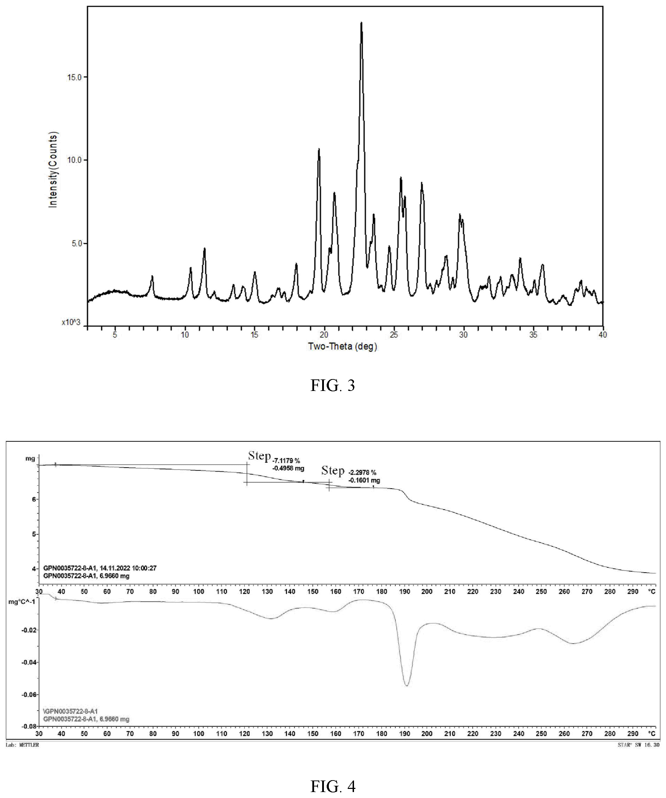

In a specific embodiment, the crystalline form A is characterized by a Cu-Kα radiation X-ray powder diffraction pattern represented by 20 values as shown in Fig. 3.

In a specific embodiment, according to thermogravimetric analysis curve, the crystalline form A exhibits a total weight loss of less than 10% (specifically 9.41%) during the process of heating to 170±2°C. Specifically, the DTG curve of the crystalline form A shows two peaks in the range from 110±2°C to 170±2°C with a total TGA weight loss of 9.41%.

In a specific embodiment, the thermogravimetric analysis curve of the crystalline form A is shown in Fig. 4.

In a specific embodiment, the differential scanning calorimetry curve of the crystalline form A shows an endothermic peak at 148.98±2°C and an exothermic peak at 185.96±2°C (indicating degradation of the crystalline form A). Specifically, the differential scanning calorimetry curve of the crystalline form A shows an endothermic peak in the range from 115.65±2°C to 163.13±2°C, with the peak at 148.98±2°C. The differential scanning calorimetry curve of the crystalline form A shows an exothermic peak in the range from 166.51±2°C to 199.38±2°C, with the peak at 185.96±2°C.

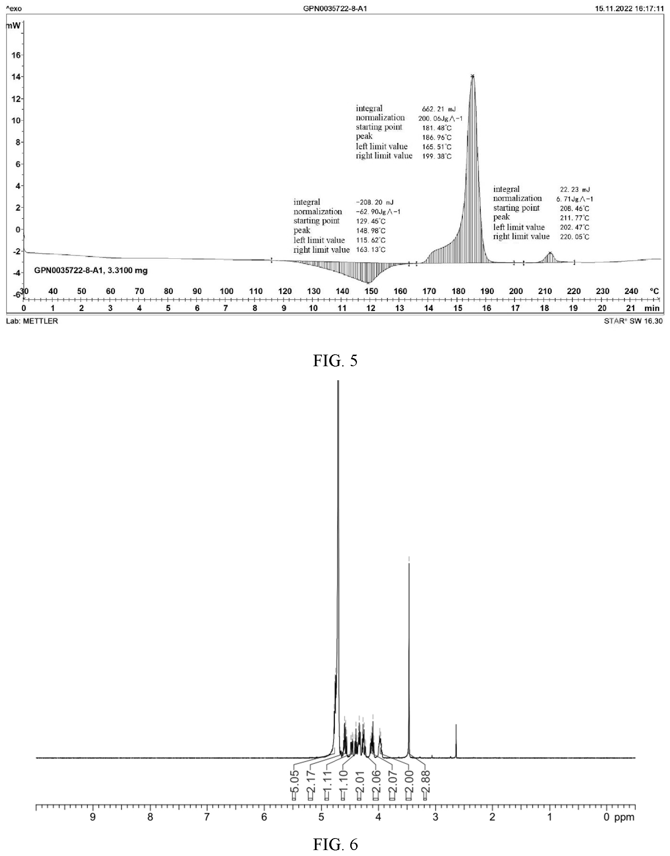

In a specific embodiment, the differential scanning calorimetry curve of the crystalline form A is shown in Fig. 5.

In a specific embodiment, the crystalline form A of the present application is a hydrate.

In a specific embodiment, the crystalline form C is characterized by a Cu-Kα radiation X-ray powder diffraction pattern with characteristic diffraction peaks present at 20 angles further comprising any one or more of 10.98±0.2°, 11.82±0.2°, 13.04±0.2°, 21.86±0.2°, 24.88±0.2°, 27.60±0.2°, and 30.10±0.2°.

In a specific embodiment, the crystalline form C is characterized by a Cu-Kα radiation X-ray powder diffraction pattern with characteristic diffraction peaks present at 20 angles further comprising any one or more of 5.78±0.2°, 16.30±0.2°, 18.62±0.2°, 19.50±0.2°, 19.84±0.2°, 22.78±0.2°, and 25.38±0.2°.

Specifically, the crystalline form C is characterized by a Cu-Kα radiation X-ray powder diffraction pattern with characteristic diffraction peaks present at 2θ angles further comprising any one or more of 10.14±0.2°, 11.38±0.2°, 14.82±0.2°, 16.94±0.2°, 24.20±0.2°, 28.00±0.2°, 28.74±0.2°, 31.86±0.2°, and 32.58±0.2°.

Specifically, the crystalline form C is characterized by a Cu-Kα radiation X-ray powder diffraction pattern with characteristic diffraction peaks present at 20 angles further comprising any one or more of 4.80±0.2°, 8.10±0.2°, 9.62±0.2°, 13.44±0.2°, 13.88±0.2°, 14.48±0.2°, 15.88±0.2°, 17.46±0.2°, 17.86±0.2°, 19.02±0.2°, 21.28±0.2°, 22.30±0.2°, 25.82±0.2°, 26.18±0.2°, 26.50±0.2°, 27.02±0.2°, 28.46±0.2°, 29.36±0.2°, 30.66±0.2°, 31.24±0.2°, 33.04±0.2°, 33.38±0.2°, 33.82±0.2°, 34.66±0.2°, 35.14±0.2°, 35.98±0.2°, 36.50±0.2°, 36.76±0.2°, 37.22±0.2°, 37.60±0.2°, 38.10±0.2°, 38.74±0.2°, and 39.54±0.2°.

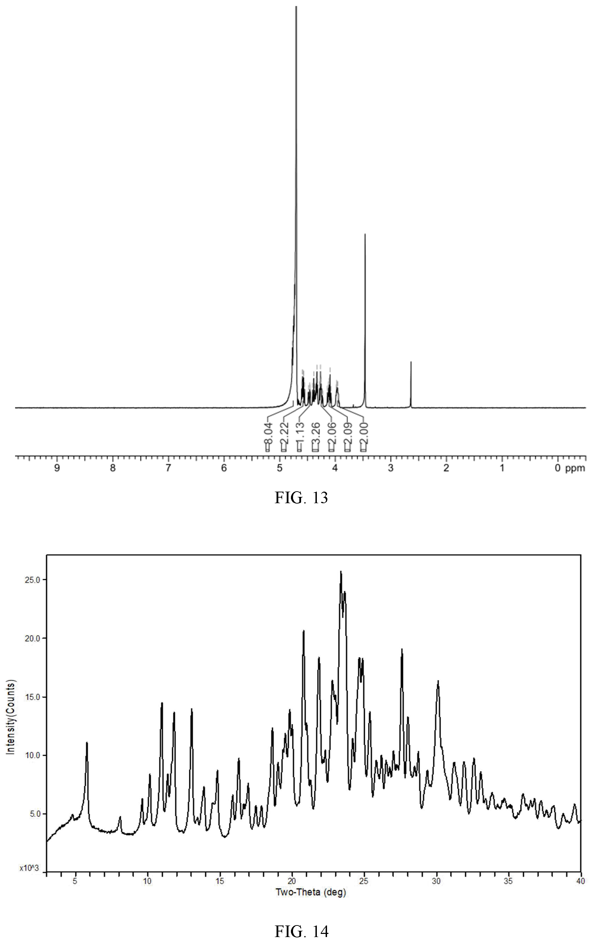

In a specific embodiment, the crystalline form C is characterized by a Cu-Kα radiation X-ray powder diffraction pattern represented by 2θ values as shown in Fig. 14.

In a specific embodiment, according to thermogravimetric analysis curve, the crystalline form C exhibits a total weight loss of less than 16% (specifically 15.42%) during the process of heating to 150±2°C. Specifically, the DTG curve of the crystalline form C shows an endothermic peak at specifically 70°C to 150°C with a total TGA weight loss of 15.42%.

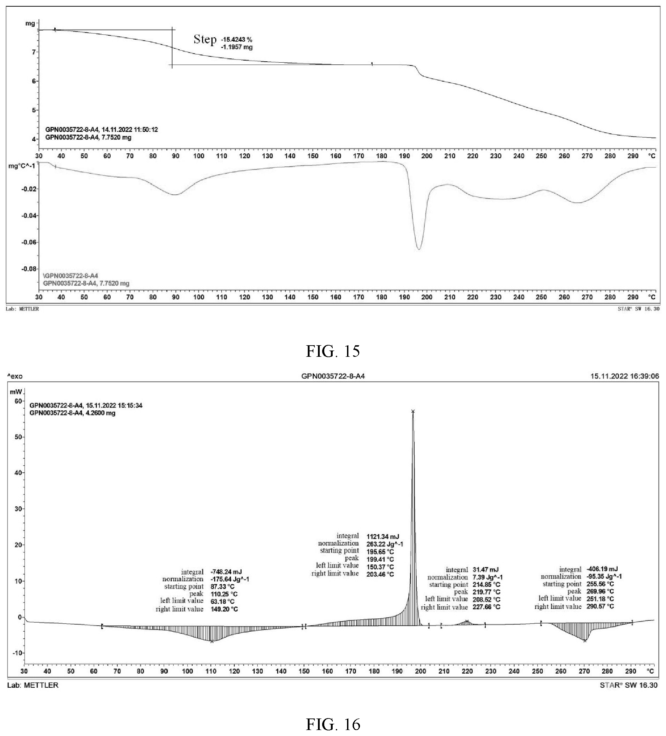

In a specific embodiment, the thermogravimetric analysis curve of the crystalline form C is shown in Fig. 15.

In a specific embodiment, the differential scanning calorimetry curve of the crystalline form C shows an endothermic peak at 110.25±2°C and an exothermic peak at 199.41±2°C (indicating possible degradation of the crystalline form C). Specifically, the differential scanning calorimetry curve of the crystalline form C shows an endothermic peak in the range from 63.18±2°C to 149.20±2°C, with the peak at 110.25±2°C. The differential scanning calorimetry curve of the crystalline form C shows an exothermic peak in the range from 150.37±2°C to 203.46±2°C, with the peak at 199.41±2°C.

In a specific embodiment, the differential scanning calorimetry curve of the crystalline form C is shown in Fig. 16.

In a specific embodiment, the crystalline form C of the present application is a hydrate crystalline form.

As used herein, the term "crude compound" refers to a substance that has a low purity and a high number of impurities and requires further purification before it can be used as an "active pharmaceutical ingredient".

As used herein, the term "purified compound" refers to a substance with increased purity compared to a "crude compound". Specifically, it refers to a substance with sufficiently high purity to meet the requirements for use as an "active pharmaceutical ingredient".

As used herein, in the field, the term "anti-solvent addition" refers to a process in which the product to be purified is dissolved in a certain good solvent, followed by the addition of an anti-solvent to induce product crystallization.

As used herein, in the field, the term "cooling crystallization" refers to a process in which the product to be purified is added to a certain solvent, heated until completely dissolved, and then cooled to induce crystallization.

As used herein, in this field, the term "slurrying purification" refers to a process in which the product to be purified is suspended in a certain solvent, slurried with stirring, and then filtered to afford the solid.

In a specific embodiment, the purification method of the compound, sodium β-O-methyl-D-cellobioside heptasulfate, can afford the crystalline form of the compound, preferably the crystalline form A.

In a specific embodiment, the C1 to C6 alcohol is selected from one or more of methanol, ethanol, and isopropanol.

In a specific embodiment, the anti-solvent addition purification method comprises the steps of dissolving the compound, sodium β-O-methyl-D-cellobioside heptasulfate, in a first solvent and slowly adding a second solvent, followed by stirring, crystallizing, and filtering to afford the purified compound.

In a specific embodiment, in the anti-solvent addition purification method, the second solvent is one or more of methanol, ethanol, isopropanol, and acetone.

In a specific embodiment, in the anti-solvent addition purification method, the volume ratio of the first solvent to the second solvent is from 1:1 to 1:20 (e.g., 1:1.5, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, 1:10, 1:10.5, 1:11, 1:11.5, 1:12, 1:12.5, 1:13, 1:13.5, 1:14, 1:14.5, 1:15, 1:15.5, 1:16, 1:16.5, 1:17, 1:17.5, 1:18, 1:18.5, 1:19, 1:19.5, or 1:20).

In a specific embodiment, the anti-solvent addition purification method may further comprise the step of adding a crystal seed.

In a specific embodiment, in the anti-solvent addition purification method, the second solvent is added in a slow manner (e.g., dropwise).

In a specific embodiment, the anti-solvent addition purification method further comprises the step of rinsing or slurrying the filter cake in the second solvent.

In a specific embodiment, the anti-solvent addition purification method further comprises the step of drying the filter cake. Specifically, the drying may be vacuum drying or freeze drying.

In a specific embodiment, the slurrying purification method comprises the steps of dispersing the compound sodium, β-O-methyl-D-cellobioside heptasulfate, to a solvent X to obtain a suspension, followed by stirring and filtering to afford a purified compound; wherein the solvent X is a mixture of a first solvent and a second solvent.

The suspension-mediated crystal transformation in crystal screening (at room temperature or 50°C) is essentially a slurrying operation.

In a specific embodiment, in the slurrying purification method, the second solvent is selected from one or more of methanol, ethanol, isopropanol, and acetone.

In a specific embodiment, the slurrying purification method may be carried out at 10 to 50°C, and further may be carried out at room temperature.

In a specific embodiment, in the slurrying purification method, the volume ratio of the first solvent to the second solvent is from 1:5 to 1:12 (e.g., 1:5, 1:5.5, 1:6, 1:6.5, 1:7, 1:7.5, 1:8, 1:8.5, 1:9, 1:9.5, 1:10, 1:10.5, 1:11, or 1:11.5).

In a specific embodiment, the slurrying purification method further comprises the step of rinsing or slurrying the filter cake in the second solvent.

In a specific embodiment, the slurrying purification method further comprises the step of drying the filter cake. Specifically, the drying may be vacuum drying or freeze drying.

In a specific embodiment, the cooling crystallization purification method comprises the steps of adding the compound, sodium β-O-methyl-D-cellobioside heptasulfate, to a mixed solvent Y and dissolved by stirring at high temperature, followed by cooling crystallization and filtering to afford a purified compound; the mixed solvent Y is a mixture of the first solvent and the second solvent.

In a specific embodiment, in the cooling crystallization purification method, the second solvent is selected from one or more of methanol, ethanol, isopropanol, and acetone.

In a specific embodiment, in the cooling crystallization purification method, the volume ratio of the first solvent to the second solvent is from 1:1 to 1:8 (e.g., 1:2, 1:2.5, 1:3, 1:3.5, 1:4, 1:4.5, 1:5, 1:5.5, 1:6, 1:6.5, 1:7, or 1:7.5).

In a specific embodiment, the cooling crystallization purification method further comprises the step of rinsing or slurrying the filter cake in the second solvent.

In a specific embodiment, the cooling crystallization purification method further comprises the step of drying the filter cake. Specifically, the drying may be vacuum drying or freeze drying.

In a specific embodiment, in the method of preparing the crystalline form C, the volume ratio of the first solvent to the second solvent is from 1:9 to 1:11 (e.g., 1:9.5, 1:10, or 1:10.5).

In a specific embodiment, the preparation of the crystalline form C may be carried out at 10°C to 60°C, further may be carried out at 10°C to 50°C, and more further may be carried out at room temperature.

As used herein, the term "active pharmaceutical ingredient"' refers to the drug substance used for further preparation or production of various formulations (especially the effective component in the formulation, also known as the active ingredient) or synthetic intermediates. It is prepared by methods such as chemical synthesis or biotechnology as powders, crystals, or other forms for pharmaceutical use, but cannot be directly administered to subjects.

In a specific embodiment, the crystalline form of the present application as described above (e.g., the crystalline form A or the crystalline form C) in the active pharmaceutical ingredient (sodium β-O-methyl-D-cellobioside heptasulfate active pharmaceutical ingredient) is from 80.0% to 100% by weight, and further may be from 90.0% to 100%, e.g., 90.0%, 91.0%, 92.0%, 93.0%, 94.0%, 95.0%, 96.0%, 97.0%, 98.0%, 99.0%, 99.1%, 99.2%, 99.3%, 99.4%, 99.5%, 99.6%, 99.7%, 99.8%, 99.9%, or 100%. In addition to crystalline forms, the active pharmaceutical ingredient may include an amorphous form and other crystalline forms of the compound, water, or other substances such as impurities or solvent residues within the limits of the quality standard.

In a specific embodiment, the crystalline form of the present application (e.g., the crystalline form A or the crystalline form C) is from 1% to 99.9% by weight of the composition. For example, the crystalline forms of the compound of the present application is 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.2%, 99.8%, or 99.9% by weight of the composition.

In a specific embodiment, the composition of the present application is a stabilized pharmaceutical composition.

In a specific embodiment, the present invention provides a composition comprising the active pharmaceutical ingredient and one or more pharmaceutically acceptable excipients.

As used herein, the term "pharmaceutically acceptable excipient" refers to an excipient that does not cause significant irritation to organisms and does not interfere with the biological activity and properties of the active ingredient (e.g., the crystalline form of the compound of the present application or the active pharmaceutical ingredient) being administered. Specifically, the pharmaceutically acceptable excipient includes, but is not limited to: a diluent, a filler, a disintegrant, a wetting agent, a lubricant, a pH modifier, a buffering agent, a colorant, a flavoring agent, a preservative, or other conventional additives.

In a specific embodiment, the composition further comprises a buffering agent. Specifically, the buffering agent includes, but is not limited to: phosphate buffers (e.g., disodium hydrogen phosphate and sodium dihydrogen phosphate buffer system), citrate buffer systems (e.g., sodium citrate/citric acid buffer system), or acetate buffers (e.g., sodium acetate/acetic acid buffer system).

The pharmaceutically acceptable excipient which forms a pharmaceutical composition with the crystalline forms of the present application may depend on the intended method of administering the pharmaceutical composition.

The crystalline forms (or the active pharmaceutical ingredient) of the present application may have systemic and/or localized activity. To this end, it may be administered in a suitable manner, for example, administration via oral, parenteral, pulmonary, nasal, sublingual, translingual, buccal, rectal, vaginal, cutaneous, transdermal, conjunctival, otic routes, or as an implant or stent. For these routes of administration, the crystalline forms of the compound of the present application can be administered in suitable administration forms.

Specifically, for oral administration, the crystalline forms (or the active pharmaceutical ingredient) of the present application may be formulated in dosage forms known in the art, which are delivered rapidly and/or in a gentle manner, the dosage forms being, for example, tablets, orally disintegrating tablets, lamellar tablets, lyophilisate, capsules (e.g., hard gelatine capsules or soft gelatine capsules), sugar-coated tablets, granules, pills, powders, emulsions, suspensions, aerosols, or solutions.

Specifically, the parenteral administration may be carried out by preventing an absorption step (e.g., intravenous, intra-arterial, intracardiac, intraspinal, or intralumbar) or including an absorption step (e.g., intramuscular, subcutaneous, intradermal, transdermal, or intraperitoneal). The administration form suitable for parenteral administration is especially an injection and infusion formulation in the form of a solution, a suspension, an emulsion, a lyophilisate, or a sterile powder.

In a specific embodiment, the composition may be a sterile powder. For use, the sterile powder is reconstituted into an injectable solution for clinical use (for example, dissolving the powder for injection in water for injection, Ringer's solution, isotonic saline, glucose solution, etc., to prepare an injectable solution). Specifically, the sterile powder may further comprise a buffering agent (the buffering agent may effectively inhibit acid hydrolysis/degradation of the compound after the sterile powder has been reconstituted into an injectable solution).

As a specific embodiment of the present invention, there is provided use of the crystalline form, the active pharmaceutical ingredient, or the composition as extracellular histone inhibitor in the preparation of a medicament for treating an extracellular histone-mediated disease.

The term "extracellular histone-mediated disease or condition" as used herein may be any of the extracellular histone-mediated diseases or conditions described in Patent NO. CN201880080079.X, which is incorporated herein by reference.

Normally, histones are found only in the nucleus, where they form nucleosomes with DNA. In disease states, histones and nucleosomes are released from dead cells into the circulation, and the released histones have direct toxic effects on cells and the endothelium, leading to endothelial dysfunction, cell death, tissue damage, platelet activation, induction of erythrocyte aggregation and cleavage, microcirculatory disturbances, and organ damage or dysfunction. Extracellular histones include, in addition to those released by lysis of tissue cells, those released by lysis of immune cells (e.g., histones in the neutrophil trapping network).

The term "extracellular histone-mediated disease or condition" generally refers to related diseases or conditions induced by the release of histones into circulation (extracellular histones), including but not limited to: (1) inflammation (e.g., acute inflammation), including inflammatory diseases, hyperinflammatory responses, inflammatory injury, pneumonia (including severe pneumonia); (2) acute organ injury or organ dysfunction (generally referring to acute organ injury or organ dysfunction caused by acute inflammation): including but not limited to acute lung injury, acute respiratory distress syndrome, acute kidney injury, acute liver injury, acute pancreatitis; (3) systemic inflammatory response, which can be triggered by infectious causes or non-infectious causes, wherein the infectious causes include bacteria, viruses, fungi, parasites, prions, etc.; and the non-infectious causes include surgery, trauma, hemorrhage, burns, acute pancreatitis, and acute kidney injury (systemic inflammatory response syndrome, SIRS); (4) Sepsis (including septic shock), generally caused by infectious causes (bacteria, viruses, fungi, parasites, prions); (5) hemostasis or vascular occlusion (mainly hemostatic or thrombotic diseases caused by extracellular histone-mediated local coagulation) e.g.: cardiovascular diseases (e.g., atherosclerosis), coagulation and thrombosis (e.g., deep vein thrombosis); (6) ischemic injury, ischemic stroke; (7) ischemia-reperfusion injury; (8) autoimmune disease states and inflammation disease states (for example, an inflammatory state during the onset of autoimmune disease), including but not limited to multiple sclerosis, hyper-inflammatory disease states, systemic lupus erythematosus, spondyloarthropathy, ankylosing spondylitis, psoriatic arthritis, reactive arthritis, enteropathic arthritis, ulcerative colitis, Crohn's disease, irritable bowel disease, rheumatoid arthritis, juvenile rheumatoid arthritis, anti-neutrophil cytoplasmic antibody associated vasculitis (such as granulomatosis with polyangiitis, eosinophilic granulomatosis with polyangiitis and microscopic polyangiitis), characterized by destruction and inflammation of small vessels, familial Mediterranean fever, amyotrophic lateral sclerosis, Sjogren's syndrome, early arthritis, viral arthritis, psoriasis, age-related organ fibrosis, idiopathic pulmonary fibrosis, juvenile diabetes (Type I), diabetes mellitus (Type 2), antiphospholipid syndrome, and (9) various central nervous system diseases such as Huntington's disease.

Those skilled in the art are aware that as medicine evolves, the criteria for diagnosing diseases evolve accordingly, and that the criteria for geographic diseases may vary from country to country. The diseases or symptoms described in this application are subject to different diagnostic criteria in various countries in practice. It should be understood that all diseases and condition categories associated with extracellular histone release are within the scope of the present application.

The embodiments or different preferred levels of embodiments described in the present application may be arbitrarily combined unless otherwise indicated.

The solutions of the present invention will be explained below in connection with examples. It will be understood by those skilled in the art that the following examples are only intended to illustrate the invention and should not be considered as limiting the scope of the invention. Where specific techniques or conditions are not indicated in the examples, follow the techniques or conditions described in the literature in the art or in accordance with the product specification. The reagents or instruments used without indication of the manufacturer are conventional products that are commercially available.

### Example

### Example 1 Preparation of the sodium β-O-methyl-D-cellobioside heptasulfate

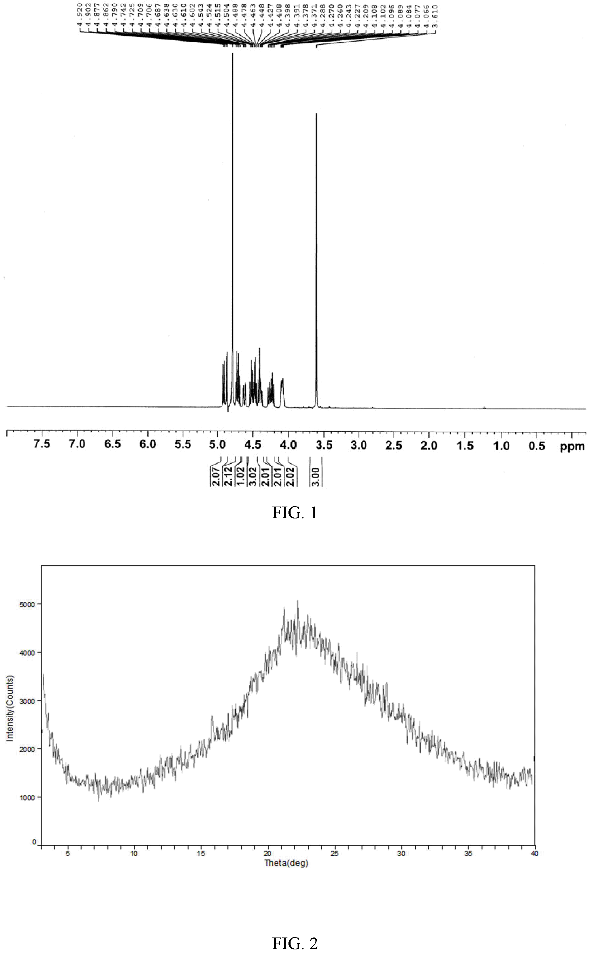

Sodium β-O-methyl-D-cellobioside heptasulfate were prepared by repeating the preparation method of Patent NO. CN201880080079.X in Example 1 (i.e., mCBS.Na in the Patent, with a purity of 90%). H¹NMR is shown in Fig. 1, and XRPD (Figure 2) is amorphous with no distinct characteristic peak.

### Example 2 Purification of the compound

### 2.1 Anti-solvent addition

The crude sodium β-O-methyl-D-cellobioside heptasulfate obtained from Example 1 was dissolved in purified water, and a second solvent was slowly added dropwise at room temperature, with continue stirring (to obtain a suspension). The suspension was filtered under suction to obtain a wet cake, followed by drying under vacuum at room temperature to afford the purified compound. The details were shown in Table 1-1.

**Table 1-1**

| Second solvent type | Purity of the purified compound CAD% |
|---|---|
| Methanol | 99.9% |
| Ethanol | 99.6% |
| Isopropanol | 99.0% |
| Acetone | 99.4% |
| Acetonitrile | 98.5% |
| Tetrahydrofuran* | 92.3% |

| | |
|---|---|
| NOTE* A white viscous substance was precipitated from the bottom during the dropwise addition of tetrahydrofuran to 1:20, which was centrifuged and blown dry to afford a solid. | |

The solution was clarified during dropwise addition of N,N-dimethylformamide and no solid was obtained.

### 2.2 Cooling Crystallization

The crude sodium β-O-methyl-D-cellobioside heptasulfate obtained from Example 1 was added to the mixed solvent shown in Table 1-2 and stirred at elevated temperature until the solution clarifies, then the solution was cooled to precipitate a white solid. The white solid was filtered under suction and dried to afford the purified compound. The details were shown in Table 1-2.

**Table 1-2**

| Mixed solvent (v: v) | Purity CAD% |
|---|---|
| Anhydrous methanol/water | 99.5% |
| Anhydrous ethanol/water | 99.2% |
| Isopropyl alcohol/water | 98.5% |
| Acetone | 99.0% |
| Acetonitrile/water | 98.5% |
| Tetrahydrofuran/water | 92.3% |
| N,N-dimethylformamide/water | 92.0% |

### 2.3 Slurrying purification

The crude sodium β-O-methyl-D-cellobioside heptasulfate obtained from Example 1 was dispersed to the mixed solvent shown in Table 1-3 to obtain a suspension, followed by stirring and filtering under suction to afford the purified compound. The details were shown in Table 1-3.

**Table 1-3**

| Mixed solvent (v: v) | Purity CAD% |
|---|---|
| Anhydrous methanol/water | 99.2% |
| Anhydrous ethanol/water | 98.8% |
| Isopropyl alcohol/water | 98.3% |
| Acetone | 98.5% |
| Acetonitrile/water | 98.0% |
| Tetrahydrofuran/water | 91.2% |

### Example 3 Screening of crystalline form

In this example, nearly 100 conditions were screened using methods such as anti-solvent addition, gas-solid diffusion, gas-liquid diffusion, slow evaporation, stirring at room temperature/50 °C, slow cooling, and polymer-induction, resulting in the obtainment of the crystalline form A, the crystalline form B, and the crystalline form C.

**3.1 Anti-solvent addition:** The starting sample was weighed into a glass vial, and a corresponding solvent was added and stirred for dissolution to obtain a clarified solution. The corresponding anti-solvent was added gradually to the above glass vial, and the experimental results were shown in Table 2-1.

**Table 2-1 The experimental results of anti-solvent addition**

| Solvent | Anti-solvent | Phenomenon | Crystalline form |
|---|---|---|---|
| Water | Anhydrous methanol | White suspension | Crystalline form A |
| | Anhydrous ethanol | Off-white solid | Crystalline form A |
| | Isopropanol | White suspension | Crystalline form A |
| | Acetone | Small amount of off-white solid | Crystalline form A |
| | Tetrahydrofuran | Very small amount of off-white solid | Amorphous form |
| | Acetonitrile | Small amount of off-white solid | Amorphous form |
| | N,N-Dimethylformamide | Clarified, no solid obtained | / |
| Toluene | Ethanol | Clarified, no solid obtained | / |
| | Methanol | Clarified, no solid obtained | / |
| | Methyl tert-butyl ether | Clarified, no solid obtained | / |
| Dimethyl sulfoxide | Ethanol | Clarified, no solid obtained | / |
| | Trichloromethane | Clarified, no solid obtained | / |
| Methylpyrrolidone | Acetone | Clarified, no solid obtained | / |
| | Ethyl acetate | Clarified, no solid obtained | / |

**3.2 Gas-solid diffusion:** The starting sample was weighed into a glass vial. A volatile solvent was charged in a large glass bottle. The above glass vial was placed in the large glass bottle for 10 days, then the solids were subjected to XRPD analysis. The experimental results showed (Table 2-2) that only amorphous form was obtained by the gas-solid diffusion method.

**Table 2-2 The experimental results of gas-solid diffusion**

| Solvent | Phenomenon | Crystalline form |
|---|---|---|
| Anhydrous methanol | Solid undissolved | Amorphous form |
| Acetone | Solid undissolved | Amorphous form |
| Ethyl acetate | Solid undissolved | Amorphous form |
| Methyl tert-butyl ether | Solid undissolved | Amorphous form |
| Tetrahydrofuran | Solid undissolved | Amorphous form |
| Acetonitrile | Solid undissolved | Amorphous form |
| Toluene | Solid undissolved | Amorphous form |
| Dichloromethane | Solid undissolved | Amorphous form |
| N,N-Dimethylformamide | Solid undissolved | Amorphous form |
| Dimethyl sulfoxide | Solid undissolved | Amorphous form |
| *n*-Heptane | Solid undissolved | Amorphous form |
| Cyclohexane | Solid undissolved | Amorphous form |
| N-methylpyrrolidone | Solid undissolved | Amorphous form |
| Anhydrous ethanol | Solid undissolved | Amorphous form |
| Water | Solid completely dissolved | Amorphous form |

**3.3 Gas-liquid diffusion:** A certain amount of starting sample was weighed into a glass vial, and a corresponding solvent was added to dissolve the starting sample by shaking at room temperature to obtain a clarified solution. Anti-solvent was charged in a large glass bottle. The above vial with an open top was placed in the large glass bottle, and the large glass bottle was tightened with a lid. The large glass bottle was allowed to stand at room temperature until a solid was precipitated. The resulting solid was characterized by XRPD. The experimental results (Table 2-3) showed that the products obtained by gas-liquid diffusion screening were amorphous.

**Table 2-3 The experimental results of gas-liquid diffusion**

| Solvent | Anti-solvent | Phenomenon | Crystalline form |
|---|---|---|---|
| | Anhydrous methanol | Small amount of solid precipitated at the bottom | Amorphous form |
| | Anhydrous ethanol | Small amount of solid precipitated at the bottom | Amorphous form |
| Water | Acetone | Small amount of solid precipitated at the bottom | Amorphous form |
| | 2-Butanone | Small amount of solid precipitated at the bottom | Amorphous form |
| | Tetrahydrofuran | Small amount of solid precipitated at the bottom | Amorphous form |
| | Acetonitrile | Small amount of solid precipitated at the bottom | Amorphous form |
| | *sec*-Butanol | Small amount of solid precipitated at the bottom | Amorphous form |
| | Methyl isobutyl ketone | Small amount of solid precipitated at the bottom | Amorphous form |

**3.4 Slow evaporation:** A certain amount of the starting sample was weighed into a glass vial, and a corresponding solvent was added for dissolution. The vial was sealed and a few pinholes were made to allow it to evaporate at 50°C. The resulting solids were subjected to XRPD analysis. The experimental results (Table 2-4) showed that the products obtained by evaporation screening were amorphous.

**Table 2-4 The experimental results of evaporation**

| Solvent | Phenomenon | Crystalline form |
|---|---|---|
| Anhydrous methanol/water | Volatilized to give a solid | Amorphous form |
| Anhydrous ethanol/water | Volatilized to give a solid | Amorphous form |
| Acetone/water | Volatilized to give a solid | Amorphous form |
| Tetrahydrofuran/water | Volatilized to give a solid | Amorphous form |
| Acetonitrile/water | Volatilized to give a solid | Amorphous form |
| N,N-Dimethylformamide | Volatilized to give an oily product | / |
| Dimethyl sulfoxide | Volatilized to give an oily product | / |
| N-methylpyrrolidone | Volatilized to give a viscous product | / |
| Toluene | Volatilized to give a solid | Amorphous form |
| Water | Volatilized to give a solid | Amorphous form |

**3.5 Cooling crystallization:** The starting material was weighed into a vial. A corresponding solvent was added and stirred at high temperature until the solution was clarified, and then subjected to cooling crystallization. The final solid obtained was characterized by XRPD and the experimental results were shown in Table 2-5.

**Table 2-5 The experimental results of cooling crystallization**

| Mixed solvent | Phenomenon | Crystalline form |
|---|---|---|
| Anhydrous methanol/water | White solid precipitated | A |
| Anhydrous ethanol/water | White solid precipitated | A |
| Isopropyl alcohol/water | White solid precipitated | A |
| Acetone/water | White solid was obtained | A |
| Tetrahydrofuran/water | White solid was obtained | Amorphous form |
| Acetonitrile/water | White solid was obtained | Amorphous form |
| N,N-dimethylformamide/water | White solid was obtained | Amorphous form |
| N,N-Dimethylformamide | An oily product was obtained | / |
| Dimethyl sulfoxide | A viscous product was obtained | / |
| N-methylpyrrolidone | A viscous product was obtained | / |
| Water | White solid was obtained | Amorphous form |

**3.6 Room-temperature suspension-mediated crystal transformation:** A certain amount of starting material was weighed into a vial, and a corresponding solvent was added with stirring at room temperature to obtain a suspension. After several days, the solid was separated by centrifugation and subjected to XRPD analysis. The experimental results were shown in Table 2-6.

**Table 2-6 The experimental results of room-temperature suspension-mediated crystal transformation**

| Solvent | Phenomenon | Crystalline form |
|---|---|---|
| Anhydrous methanol/water | Suspension | A |
| Anhydrous ethanol/water | | A |
| Isopropyl alcohol/water | | A |
| Acetone/water | | A |
| 1,4-Dioxane/water | | C |
| Acetonitrile/water | | Amorphous form |
| N,N-dimethylformamide/water | Clarified viscous liquid | B |
| Anhydrous ethanol/toluene | Suspension | Amorphous form |
| Anhydrous ethanol/dimethyl sulfoxide | | Amorphous form |
| N-methylpyrrolidone/acetone | | Amorphous form |
| Anhydrous methanol | | Amorphous form |
| Acetone | | Amorphous form |
| Ethyl acetate | | Amorphous form |
| Tetrahydrofuran | | Amorphous form |
| Methyl tert-butyl ether | | Amorphous form |
| Cyclohexane | | Amorphous form |
| Water | Clarified viscous liquid | Amorphous form |

**3.7 50°C suspension-mediated crystal transformation:** A certain amount of starting material was weighed into a vial, and a corresponding solvent was added with stirring at 50°C to obtain a suspension. After several days, the solid was separated by centrifugation and subjected to XRPD analysis. The experimental results were shown in Table 2-7.

**Table 2-7 The experimental results of 50°C suspension-mediated crystal transformation**

| Solvent | Phenomenon | Crystalline form |
|---|---|---|
| Anhydrous methanol/water | Suspension | A |
| Anhydrous ethanol/water | | A |
| Isopropyl alcohol/water | | A |
| Acetone/water | | A |
| Acetonitrile/water | | Amorphous form |
| 1,4-Dioxane/water | | C |
| N,N-dimethylformamide/water | Clarified viscous liquid | B |
| Anhydrous ethanol/toluene | Suspension | Amorphous form |
| Anhydrous ethanol/dimethyl sulfoxide | | Amorphous form |
| N-methylpyrrolidone/acetone | | Amorphous form |
| Anhydrous methanol | | Amorphous form |
| Acetone | | Amorphous form |
| Ethyl acetate | | Amorphous form |
| Tetrahydrofuran | | Amorphous form |
| Methyl tert-butyl ether | | Amorphous form |
| Cyclohexane | | Amorphous form |
| Water | Clarified viscous liquid | Amorphous form |

**3.8 Polymer induction:** A certain amount of starting material was weighed into a vial, and a corresponding solvent was added for dissolution. PVP K30 was added to the filtrate and allowed to evaporate at room temperature. The final resulting solid was characterized by XRPD. The experimental results (Table 2-8) showed that most of the products obtained by polymer induction were amorphous, and only evaporation in aqueous solution obtained the crystalline form A.

**Table 2-8 Polymer Induction**

| Solvent | Phenomenon | Crystalline form |
|---|---|---|
| Anhydrous methanol/water | Clarified | Amorphous form |
| Anhydrous ethanol/water | Clarified | Amorphous form |
| Acetone/water | Clarified | Amorphous form |
| Tetrahydrofuran/water | Clarified | Amorphous form |
| Acetonitrile/water | Clarified | Amorphous form |
| Toluene | Clarified | Amorphous form |
| Water | Clarified | A |

### Example 4 Identification of crystalline forms

X-ray powder diffraction data of the samples were collected under ambient conditions using a Bruker D8 model X-ray powder diffractometer with an X-ray emitter power of 300 W. The step size was 20 = 0.02°, the voltage was 30 kV and the current was 10 mA. The X-ray tube used a Cu target (Kα), with a Kα2/Kα1 intensity ratio of 0.50 (1.54439 Å / 1.5406 Å).

Thermogravimetric analysis (TGA): Thermogravimetric data of the samples were collected using a Mettler TGA 2 thermogravimeter. A few milligrams of the sample were placed in an alumina crucible and heated from room temperature to the target temperature at a heating rate of 10 °C/min under the protection of N₂ at a flow rate of 50 mL/min.

Differential Scanning Calorimetry (DSC): The thermal data of the samples were collected using a Mettler Differential Scanning Calorimeter DSC 3. A few milligrams of the sample were placed in a Tzero aluminum pan and sealed with a Tzero sealing cap. The pan was heated at a heating rate of 10 °C/min under the protection of N₂ at a flow rate of 50 mL/min.

### 4.1 Crystalline form A

The XRPD pattern of the crystalline form A was shown in Fig. 3, which showed a better peak shape, indicating higher crystallinity, and the detailed data were shown in Table 3 below.

The TGA-DTG and DSC curves of the crystalline form A were shown in Figs. 4 and 5, respectively. As can be seen, the DTG curve showed two peaks in the range from 110 °C to 170 °C, with a total mass loss of 9.41% observed in the TGA. Correspondingly, the DSC curve showed an endothermic peak at 148.98°C. ¹H NMR spectrum displays no solvent peak for methanol or ethanol (the crystalline form A was obtained by slurrying in methanol/water or ethanol/water), indicating that the crystalline form A is a hydrate.

**Table 3. XRPD diffraction peak data for the crystalline form A**

| Diffraction angle [°2θ] | d value [Å] | Relative intensity [%] (Height) |
|---|---|---|
| 7.63 | 11.5761 | 8.8 |
| 10.40 | 8.4993 | 12.4 |
| 11.38 | 7.7691 | 20.0 |
| 12.08 | 7.3206 | 3.1 |
| 13.45 | 6.5775 | 6.6 |
| 14.12 | 6.2669 | 5.8 |
| 14.98 | 5.9090 | 12.0 |
| 16.27 | 5.4431 | 2.9 |
| 16.75 | 5.2886 | 5.4 |
| 17.11 | 5.1779 | 3.5 |
| 17.97 | 4.9321 | 14.4 |
| 19.60 | 4.5255 | 57.0 |
| 20.33 | 4.3645 | 17.4 |
| 20.69 | 4.2894 | 39.6 |
| 22.32 | 3.9796 | 43.9 |
| 22.64 | 3.9242 | 100.0 |
| 23.51 | 3.7809 | 24.8 |
| 24.61 | 3.6143 | 17.6 |
| 25.45 | 3.4969 | 45.5 |
| 25.74 | 3.4582 | 38.6 |
| 26.99 | 3.3009 | 43.6 |
| 27.58 | 3.2315 | 3.6 |
| 28.04 | 3.1796 | 4.7 |
| 28.75 | 3.1026 | 13.6 |
| 29.22 | 3.0539 | 4.1 |
| 29.70 | 3.0055 | 31.8 |
| 31.18 | 2.8661 | 5.9 |
| 31.80 | 2.8117 | 9.2 |
| 32.62 | 2.7428 | 7.7 |
| 33.05 | 2.7079 | 2.4 |
| 33.41 | 2.6797 | 6.4 |
| 34.00 | 2.6346 | 14.0 |
| 35.03 | 2.5595 | 6.7 |
| 35.63 | 2.5177 | 14.0 |
| 37.12 | 2.4200 | 3.6 |
| 38.04 | 2.3636 | 5.1 |
| 38.40 | 2.3423 | 7.1 |
| 38.77 | 2.3207 | 6.1 |
| 39.33 | 2.2889 | 4.4 |

### 4.2 Crystalline form B

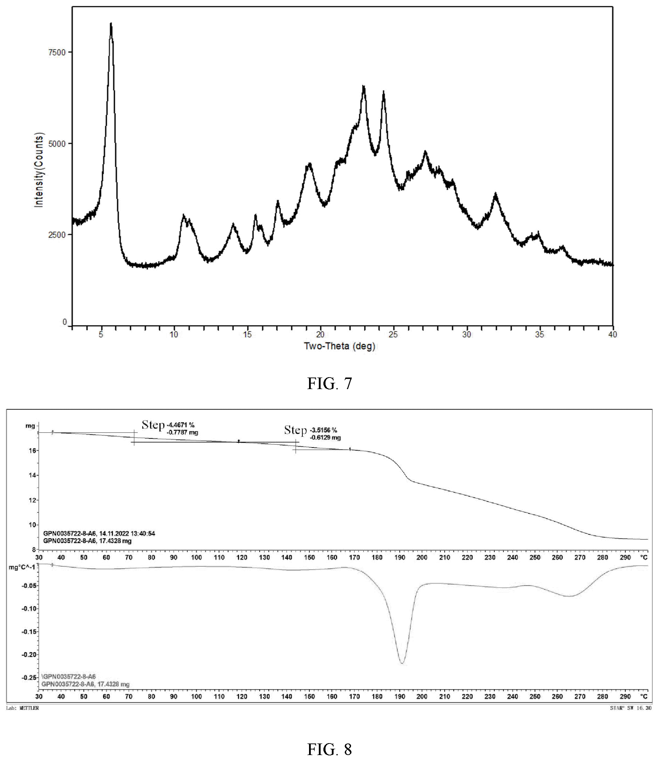

The XRPD pattern of the crystalline form B was shown in Fig. 7, and the detailed data were shown in Table 4 below.

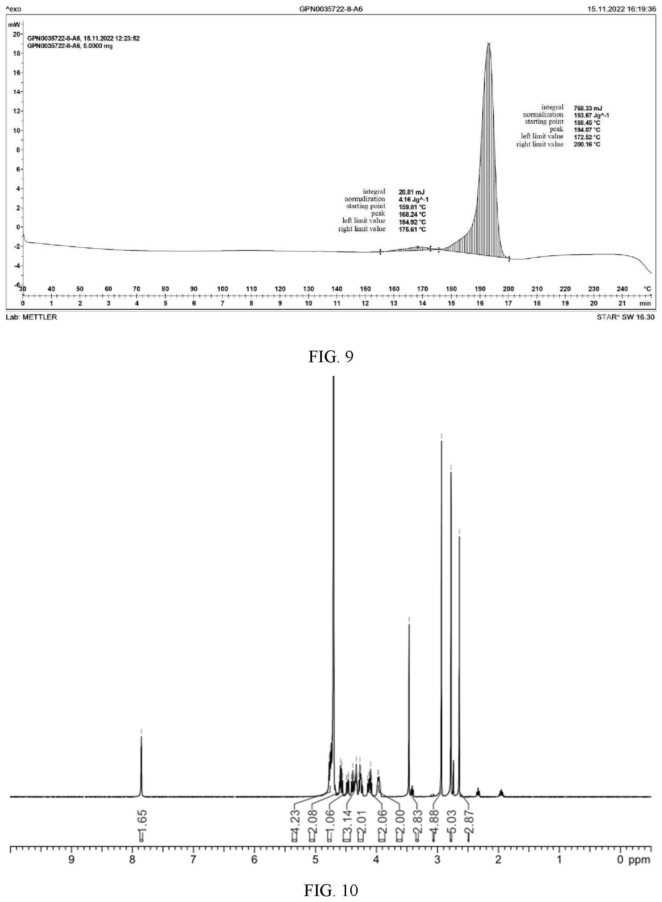

The TGA-DTG and DSC curves of the crystalline form B were shown in Figs. 8 and 9 respectively, showing that there was no distinct characteristic peak in the DTG curve prior to decomposition, and no endothermic peak was observed in the corresponding DSC curve. This confirmed that the crystalline form B was an anhydrous form. The total mass loss in TGA was 7.98%. After H¹NMR characterization, the H¹NMR spectrum was consistent with the starting compound, and the DMF solvent peak appeared in the graph (the crystalline form B was obtained by DMF/water slurrying), indicating that the crystalline form B contained DMF solvent, and the combination of the DSC and TGA curves showed that the TGA loss of gravity step was flat and there was no obvious desolventization peak in DSC, so the crystalline form B should contain free DMF solvent (due to the high boiling point of DMF, some of it was left in the solid when drying).

**Table 4. XRPD diffraction peak data for the crystalline form B**

| Diffraction angle [°2θ] | d value [Å] | Relative intensity [%] (Height) |
|---|---|---|
| 5.68 | 15.5465 | 100 |
| 10.57 | 8.3623 | 15.4 |
| 11.01 | 8.0294 | 18.5 |
| 14.01 | 6.3161 | 14.4 |
| 14.30 | 6.1891 | 8.7 |
| 15.55 | 5.6939 | 15.9 |
| 15.92 | 5.5624 | 9.8 |
| 17.02 | 5.2051 | 14.5 |
| 19.29 | 4.5974 | 21.4 |
| 21.33 | 4.1624 | 6.5 |
| 22.30 | 3.9832 | 10.7 |
| 22.98 | 3.8669 | 29.6 |
| 24.29 | 3.6613 | 35.4 |
| 26.69 | 3.3372 | 6 |
| 27.18 | 3.2781 | 10.9 |
| 28.16 | 3.1659 | 4.3 |
| 29.07 | 3.069 | 6.2 |
| 31.94 | 2.7997 | 16 |
| 34.14 | 2.624 | 3.3 |
| 34.31 | 2.6114 | 5 |
| 34.85 | 2.5722 | 7.7 |
| 36.57 | 2.4552 | 5.2 |

### 4.3 Crystalline form C

The ¹H NMR spectrum of the crystalline form C was shown in Fig. 13, and the XRPD pattern of the crystalline form C was shown in Fig. 14. The detailed data were shown in Table 5 below.

The TGA-DTG and DSC curves of the crystalline form C were shown in Figs. 15 and 16 respectively. The DTG curve of the crystalline form C showed one peak in the range from 70 °C to 120 °C, with a total mass loss of 15.42% observed in the TGA. Correspondingly, the DSC curve showed an endothermic peak at 110.25°C. ¹H NMR spectrum displays no solvent peak for dioxane (the crystalline form C was obtained by slurrying in dioxane/water), indicating that the crystalline form C is a hydrate.

**Table 5. XRPD diffraction peak data for the crystalline form C**

| Diffraction angle [°2θ] | d value [Å] | Relative intensity [%] (Height) |
|---|---|---|
| 4.80 | 18.4056 | 3.0 |
| 5.78 | 15.2741 | 38.7 |
| 8.10 | 10.9112 | 7.8 |
| 9.62 | 9.1889 | 14.7 |
| 10.14 | 8.7157 | 24.5 |
| 10.98 | 8.0540 | 57.0 |
| 11.38 | 7.7696 | 25.5 |
| 11.82 | 7.4814 | 51.0 |
| 13.04 | 6.7840 | 57.3 |
| 13.44 | 6.5839 | 5.9 |
| 13.88 | 6.3750 | 18.8 |
| 14.48 | 6.1117 | 13.3 |
| 14.82 | 5.9733 | 29.6 |
| 15.88 | 5.5776 | 12.2 |
| 16.30 | 5.4341 | 34.7 |
| 16.94 | 5.2296 | 20.0 |
| 17.46 | 5.0745 | 10.4 |
| 17.86 | 4.9621 | 9.8 |
| 18.62 | 4.7617 | 37.3 |
| 19.02 | 4.6633 | 13.0 |
| 19.50 | 4.5483 | 32.9 |
| 19.84 | 4.4719 | 41.6 |
| 20.80 | 4.2671 | 82.3 |
| 21.28 | 4.1719 | 12.5 |
| 21.86 | 4.0625 | 65.1 |
| 22.30 | 3.9837 | 15.5 |
| 22.78 | 3.9004 | 31.0 |
| 23.38 | 3.8016 | 100.0 |
| 23.64 | 3.7604 | 86.7 |
| 24.20 | 3.6747 | 21.5 |
| 24.88 | 3.5758 | 61.9 |
| 25.38 | 3.5064 | 33.7 |
| 25.82 | 3.4476 | 13.7 |
| 26.18 | 3.4010 | 14.2 |
| 26.50 | 3.3607 | 8.9 |
| 27.02 | 3.2974 | 12.9 |
| 27.60 | 3.2293 | 58.0 |
| 28.00 | 3.1838 | 28.2 |
| 28.46 | 3.1333 | 11.9 |
| 28.74 | 3.1038 | 23.0 |
| 29.36 | 3.0399 | 10.9 |
| 30.10 | 2.9665 | 55.2 |
| 30.66 | 2.9137 | 7.9 |
| 31.24 | 2.8609 | 18.2 |
| 31.86 | 2.8063 | 21.1 |
| 32.58 | 2.7461 | 25.2 |
| 33.04 | 2.7088 | 18.1 |
| 33.38 | 2.6820 | 3.9 |
| 33.82 | 2.6481 | 7.4 |
| 34.66 | 2.5857 | 6.5 |
| 35.14 | 2.5517 | 4.8 |
| 35.98 | 2.4941 | 11.1 |
| 36.50 | 2.4596 | 7.9 |
| 36.76 | 2.4428 | 8.3 |
| 37.22 | 2.4137 | 8.0 |
| 37.60 | 2.3901 | 5.6 |
| 38.10 | 2.3600 | 8.0 |
| 38.74 | 2.3225 | 5.8 |
| 39.54 | 2.2773 | 9.0 |

### Example 5 Scaled-up preparation of the crystalline form A

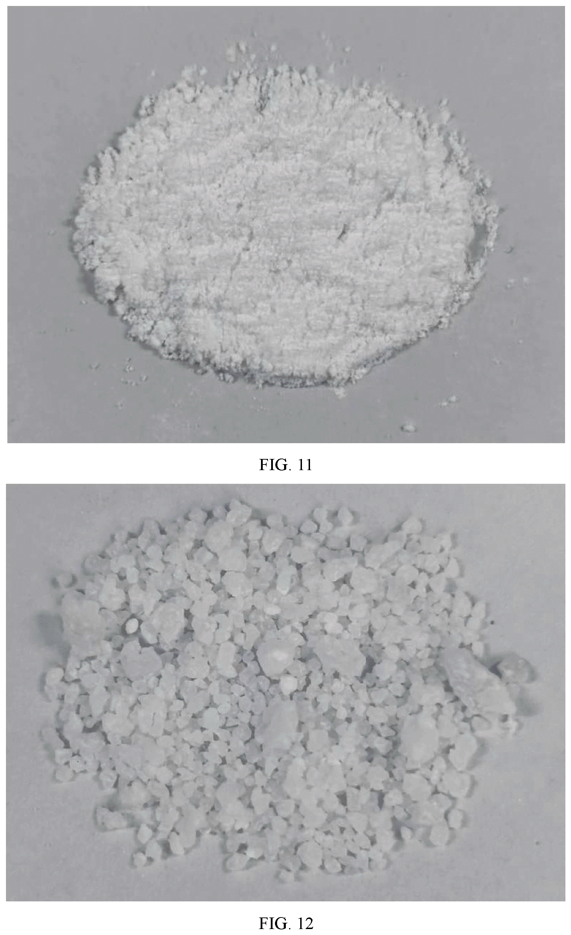

50 g of amorphous sodium β-O-methyl-D-cellobioside heptasulfate prepared according to Example 1 was dissolved in purified water (3 to 5 times the weight of the compound). Methanol (2 to 10 times the volume of purified water) was slowly added dropwise at room temperature. During the addition, the crystal seeds were introduced, and the mixture was aged for half an hour. The remaining methanol was then continued to be added dropwise, followed by further stirring. The resulting wet filter cake was collected by suction filtration. The wet cake was rinsed or slurried in methanol, subjected to suction filtration, and vacuum-dried at room temperature to obtain a white powder solid. XRPD analysis confirmed the formation of the crystalline form A. Upon scale-up across multiple batches, the crystalline form A demonstrated high crystallinity and excellent properties (a white powder, Fig. 11). Moreover, crystallization to the crystalline form A effectively enhanced the purity of the compound, with all purities of 99.8% or more and yields of 90% or more.

### Example 6 Preparation of the crystalline form B

Using N,N-dimethylformamide as an anti-solvent, methods such as anti-solvent addition, cooling crystallization, and slurrying purification were all unable to effectively purify the crude sodium β-O-methyl-D-cellobioside heptasulfate.

The purified amorphous sodium β-O-methyl-D-cellobioside heptasulfate (with a purity of 99.8% or more) was stirred in N,N-dimethylformamide/water to obtain the crystalline form B, which exhibited a waxy solid appearance (Fig. 12) and low crystallinity. The preparation process required the use of N,N-dimethylformamide, which had a high boiling point, leading to severe solvent residue that was difficult to remove. The preparation process was unsuitable for industrial scale-up and failed to effectively enhance the purity of the active pharmaceutical ingredient.

### Example 7 Scaled-up preparation of the crystalline form C

20 g of the amorphous sodium β-O-methyl-D-cellobioside heptasulfate prepared according to Example 1 was weighed into a glass vial. The corresponding solvents as shown in Table 6 were added, and the mixture was stirred magnetically at room temperature. The solid was separated by centrifugation and dried, followed by XRPD analysis, indicating that the amorphous form underwent crystal transformation to obtain the crystalline form C in 1,4-dioxane/water, and no further crystal transformation was observed after 6 days. The amorphous form was stirred in N,N-dimethylformamide/water, resulting in the crystalline form B, which exhibited low crystallinity. The preparation process required the use of N,N-dimethylformamide, which had a high boiling point, leading to severe solvent residue that was difficult to remove. The crystalline form B cannot be used as the active pharmaceutical ingredient for drugs.

**Table 6**

| Solvent | Crystalline form after 3 days | Crystalline form after 6 days |
|---|---|---|
| 1,4-Dioxane/water | C | C |
| N,N-dimethylformamide/water | B+ Amorphous | B+ Amorphous |

### Example 7 Crystal transformation experiment (solid suspension experiment)

The starting sample was weighed into a vial, and the corresponding solvent was added according to Table 7 and stirred magnetically at room temperature. After 3 days, the solid was separated by centrifugation and subjected to XRPD analysis following drying. The experimental results showed that the crystalline form A remained unchanged after stirring in different solvents; the crystalline form B did not undergo crystal transformation only in DMF/water and underwent crystal transformation to the crystalline form A in both methanol/water and ethanol/water. Therefore, the crystalline form A is stable in a wide range of solvents with good reproducibility and convenient preparation process and is the thermodynamically most stable crystalline form.

**Table 7 The experimental results of crystal transformation**

| Starting crystalline form | Solvent | Phenomenon | Crystalline form after crystal transformation |
|---|---|---|---|
| A | Anhydrous methanol/water | Suspension | A |
| B | | Suspension | A |
| A | Anhydrous ethanol/water | Suspension | A |
| B | | Suspension | A |
| A | N,N-dimethylformamide/water | Suspension | A |
| B | | Suspension | B |

### Example 8 Stability experiment

An influencing factor test was conducted on crystalline form samples, and the experimental content and results were shown in Table 8. For the crystalline form B, the total impurities content began to increase significantly after 10 days at 40°C, and the solid color changed from white to gray. The total impurities content of amorphous form significantly increased at 40°C, especially after 30 days, when the total impurities content was very high, and the appearance turned gray. In contrast, the crystalline form A and the crystalline form C can be left stable for at least 30 days at 40°C under light (5000 lux) without any change in appearance.

**Table 8**

| Placement conditions | Crystalline form A | | Crystalline form B | | Crystalline form C | | Amorphous form | |
|---|---|---|---|---|---|---|---|---|
| | Crystalline form | Total impurities | Crystalline form | Total impurities | Crystalline form | Total impurities | Crystalline form | Total impurities |
| Day 0 | A | 0.13% | B | 0.15% | C | 0.13% | Amorphous form | 0.14% |
| 40°C/75% for 5 days | A | 0.13% | B | 0.17% | C | 0.13% | Amorphous form | 0.25% |
| 40°C/75% for 10 days | A | 0.13% | B | 0.57% | C | 0.13% | Amorphous form | 0.99% |
| 40°C/75% for 30 days | A | 0.14% | B | 1.26% | C | 0.14% | Amorphous form | 4.79% |
| Light/20°C for 5 days | A | 0.08% | B | 0.15% | C | 0.09% | Amorphous form | 0.14% |
| Light/20°C for 10 days | A | 0.12% | B | 0.14% | C | 0.12% | Amorphous form | 0.15% |
| Light/20°C for 30 days | A | 0.14% | B | 0.16% | C | 0.13% | Amorphous form | 0.16% |

### Example 9 Accelerated stability study

Accelerated stability study was conducted on crystalline form samples, and the experimental content and results were shown in Table 9. It can be observed that the amorphous form was more unstable at 60°C, with the compound content decreasing to only 43.2% after 30 days. The impurities in the crystalline form B increased more significantly at 60°C, and the solid color changed from white to gray. Under high humidity conditions, both the crystalline form B and the amorphous form were transformed to the crystalline form C. The crystalline form C showed good stability advantages under high temperature, high humidity, and light conditions with no change in appearance.

**Table 9 Stability tests**

| Placement conditions | Crystalline form C | | Crystalline form B | | Amorphous form | |
|---|---|---|---|---|---|---|
| | Crystalline form | Total impurities | Crystalline form | Total impurities | Crystalline form | Total impurities |
| 60 °C for 5 days | C | 0.12% | B | 0.17% | Amorphous form | 1.25% |
| 60°C for 10 days | C | 0.11% | B | 2.08% | Amorphous form | 4.80% |
| 60°C for 30 days | C | 0.15% | B | 5.11% | Amorphous form | * (only 43.2% of compound CAD% content) |
| High humidity 75%/room temperature for 5 days | C | 0.12% | C | 0.12% | C | 0.11% |
| High humidity 75%/room temperature for 10 days | C | 0.09% | C | 0.12% | C | 0.10% |
| High humidity 75%/room temperature for 30 days | C | 0.12% | C | 0.10% | C | 0.12% |

### Example 10 Stability test at ambient temperature

Long-term stability tests under ambient temperature (25°C) were conducted on the crystalline form A sample. The results (Table 10) showed that the total content of related substances of the crystalline form A remained essentially unchanged after 6 months of storage at ambient temperature, indicating that it can be stably stored at ambient temperature for at least 6 months. Compared to amorphous form that need to be stored at low temperatures, the crystalline form A somewhat relaxes the limitation on the storage temperatures for active pharmaceutical ingredient.

**Table 10**

| | 0 | 1 month | 2 months | 3 months | 6 months |
|---|---|---|---|---|---|
| Related substances (Total impurities) | 0.10% | 0.10% | 0.11% | 0.13% | 0.13% |

### Example 11 Flowability test

The flowability of the crystalline forms was investigated using the compressibility index. The bulk density and tapped density were measured separately, and then the compressibility index was calculated according to the following formula: Compressibility index (%) = (Tapped density - Bulk density) / Tapped density × 100%. The results were shown in Table 11:

**Table 11 The results of flowability test**

| Crystalline form | Bulk density (g/cm³) | Tapped density (g/cm³) | Compressibility factor (%) |
|---|---|---|---|
| Crystalline form A | 0.34 | 0.46 | 26.00 |
| Crystalline form B | 0.30 | 0.52 | 42.31 |
| Amorphous form (Example 1) | 0.25 | 0.42 | 40.77 |

The results indicated that the compressibility index of the crystalline form A of the present invention was 26.00%, while those of the crystalline form B and the amorphous form were 42.31% and 40.77% respectively, demonstrating that the crystalline form A of the present invention possessed better flowability compared to the crystalline form B and the amorphous form, making it more suitable for formulation preparation.

### Example 12 Investigation of Pressure Stability

After pressure of 50, 100, 150, and 200N was applied to the crystalline form A and the crystalline form B, respectively for tableting, whether crystalline form change occurred was observed via XRPD. The results showed that the crystalline form of the crystalline form A did not change after tableting, while the amorphous form appeared in the crystalline form B.

### Example 13 Tablet Preparation - Direct Compression Process

Tablet preparation was carried out by direct compression process according to the composition of formulations in Table 12.

Premixing: first, the active pharmaceutical ingredient, mannitol, cross-linked sodium carboxymethyl cellulose were loaded into the three-dimensional motion mixer to mix 10min.

Final blending: A portion of the aforementioned granules was taken, mixed with magnesium stearate, and passed through a 40-mesh sieve. The mixture was then loaded into a three-dimensional mixer and blended for 5 minutes.

Tablet compression: The finally blended granules were added to the hopper of the tablet compressor. The lower discharge baffle was opened, and the granule filling in the feeder was observed through the sight glass until sufficient. The fill weight was then adjusted according to the tablet weight range to meet the required tablet weight. Subsequently, the tablet compression was controlled to adjust the average tablet hardness to within the target range.

**Table 12**

| Material Name | Formulation composition (mg) | Percentage | Role |
|---|---|---|---|
| Active pharmaceutical ingredient | 300 | 60.00% | API |
| Mannitol | 155 | 31.00% | Filler |
| Cross-linked sodium carboxymethyl cellulose | 40 | 8.00% | Disintegrant |
| Magnesium stearate | 5 | 1.00% | Lubricant |
| Total weight | 500 | 100.00% | / |

The crystalline form A can be directly compressed for tablet preparation, with good tableting effect without problems such as loose or broken tablets. The tablet weight variation meets requirements, and the tablets exhibit a dissolution behavior of 100% (pH 6.8) with complete dissolution achieved within 20 minutes.

The crystalline form B and the amorphous form had poor flowability. The applicant attempted to add colloidal silicon dioxide (0.5% to 3%) to improve flowability, and adjusted the types and amounts of excipients, but the tableting effect was not ideal, with problems such as prone to capping, tablet weight easily exceeding the ±5% target tablet weight range, and sticking to the punch and die during tableting.

### Example 14 Dry granulation process

Tablets were prepared using the dry granulation process for the crystalline form C, the crystalline form B, and the amorphous form, respectively according to the formulation composition in Table 13.
1. Premixing: First, the internally added active pharmaceutical ingredient, crospovidone, microcrystalline cellulose, and colloidal silicon dioxide were loaded into a three-dimensional motion mixer and mixed for 10 minutes. Then, magnesium stearate (internal addition) was added and mixed for 2 minutes.
2. Dry granulation: The above materials were subjected to dry granulation. When granulating, a suitable sieve was selected, the roller gap was adjusted, and the feed speed was controlled.
3. Final blending: The externally added crospovidone and colloidal silicon dioxide were weighed, mixed and passed through a 40-mesh sieve, then were added to a three-dimensional mixer and mixed at 10 rpm for 10 minutes; subsequently, the (external addition) magnesium stearate was added and mixed at 10 rpm for 3 minutes.
4. Tablet compression: The granules were added to the hopper. The lower discharge baffle was opened, and the granule filling in the feeder was observed through the sight glass until sufficient. The fill weight was then adjusted according to the tablet weight range to meet the required tablet weight. Subsequently, the tablet compression was controlled to adjust the average tablet hardness to within the target range, and a rotational speed of 10 to 14 rpm was chosen for tableting.

**Table 13**

| Material Name | Formulation composition (mg) | Percentage | Role |
|---|---|---|---|
| Internally added material | | | |
| Active pharmaceutical ingredient | 300 | 60.00% | API |
| Microcrystalline cellulose (M101LM) | 128.45 | 25.69% | Filler |
| Crospovidone | 47.05 | 9.41% | Disintegrant |
| Colloidal silicon dioxide | 2.6 | 0.52% | Flowing aid |
| Magnesium stearate | 2.6 | 0.52% | Lubricant |

| Externally added material | | | |
|---|---|---|---|
| Crospovidone | 13.35 | 2.67% | Disintegrant |
| Colloidal silicon dioxide | 3.35 | 0.67% | Flowing aid |
| Magnesium stearate | 2.6 | 0.52% | Lubricant |
| Total weight | 500 | 100.00% | / |

The crystalline form C exhibited good tableting effect, without problems such as loose or broken tablets. The tablet weight variation meets requirements, and the tablets exhibit a dissolution behavior of 100% (pH 6.8) with complete dissolution achieved within 60 minutes.

The crystalline form B and the amorphous form had problems such as poor tablet friability, prone to capping, and tablet weight easily exceeding the ±5% target tablet weight range, and these problems were not effectively resolved by adjusting other formulation composition. From the perspective of dissolution rate, the tablets of the amorphous form prepared by dry granulation exhibited a significantly slower dissolution rate compared to those of the crystalline form C prepared by the dry granulation, with the dissolution reaching only 94% (achieving 94% only after 120 minutes). Moreover, the dissolution rate of the crystalline form B was even slower than that of the crystalline form C, requiring nearly 120 minutes to achieve complete dissolution.

Although embodiments of the present invention have been shown and described above, it is to be understood that the above embodiments are exemplary and are not to be construed as a limitation of the present invention, and that one of ordinary skill in the art may make changes, modifications, substitutions, and variations of the above embodiments within the scope of the present invention.

## Claims

1. A crystalline form of the compound, sodium β-O-methyl-D-cellobioside heptasulfate, **characterized in that** it is selected from one of crystalline form A and crystalline form C, or a mixture thereof; wherein,
the crystalline form A is **characterized by** a Cu-Kα radiation X-ray powder diffraction pattern with characteristic diffraction peaks present at 20 angles comprising19.60±0.2°, 22.64±0.2°, and 25.45±0.2°; and
the crystalline form C is **characterized by** a Cu-Kα radiation X-ray powder diffraction pattern with characteristic diffraction peaks present at 20 angles comprising 20.80±0.2°, 23.38±0.2°, and 23.64±0.2°.

2. The crystalline form according to claim 1, **characterized in that**,
the crystalline form A is **characterized by** a Cu-Kα radiation X-ray powder diffraction pattern with characteristic diffraction peaks present at 20 angles further comprising any one or more of 20.69±0.2°, 22.32±0.2°, 25.74±0.2°, 26.99±0.2°, and 29.70±0.2°; and/or
the crystalline form C is **characterized by** a Cu-Kα radiation X-ray powder diffraction pattern with characteristic diffraction peaks present at 2θ angles further comprising any one or more of 10.98±0.2°, 11.82±0.2°, 13.04±0.2°, 21.86±0.2°, 24.88±0.2°, 27.60±0.2°, and 30.10±0.2°.

3. The crystalline form according to claim 1, **characterized in that**,
the crystalline form A is **characterized by** a Cu-Kα radiation X-ray powder diffraction pattern with characteristic diffraction peaks present at 20 angles further comprising any one or more of 10.40±0.2°, 11.38±0.2°, 14.98±0.2°, 17.97±0.2°, 20.33±0.2°, 23.51±0.2°, 24.61±0.2°, 28.75±0.2°, 34.00±0.2°, and 35.63±0.2°; and/or
the crystalline form C is **characterized by** a Cu-Kα radiation X-ray powder diffraction pattern with characteristic diffraction peaks present at 2θ angles further comprising any one or more of 5.78±0.2°, 16.30±0.2°, 18.62±0.2°, 19.50±0.2°, 19.84±0.2°, 22.78±0.2°, and 25.38±0.2°.

4. The crystalline form according to claim 1, **characterized in that**,
the crystalline form A is **characterized by** a Cu-Kα radiation X-ray powder diffraction pattern represented by 20 values as shown in Fig. 3; and/or
the crystalline form C is **characterized by** a Cu-Kα radiation X-ray powder diffraction pattern represented by 20 values as shown in Fig. 14.

5. The crystalline form according to claim 1, **characterized in that**,
the differential scanning calorimetry curve of the crystalline form A shows an endothermic peak at 148.98±2°C and an exothermic peak at 185.96±2°C; and/or
according to thermogravimetric analysis curve, the crystalline form A exhibits a total weight loss of less than 10% during the process of heating to 170±2°C.

6. The crystalline form according to claim 1, **characterized in that**,
the differential scanning calorimetry curve of the crystalline form C shows an endothermic peak at 110.25±2°C and an exothermic peak at 199.41±2°C; and/or
according to thermogravimetric analysis curve, the crystalline form C exhibits a total weight loss of less than 16% during the process of heating to 150±2°C.

7. The crystalline form according to claim 1, **characterized in that**, the crystalline form A is a hydrate crystalline form; and/or, the crystalline form C is a hydrate crystalline form.

8. An active pharmaceutical ingredient, **characterized in that** it comprises the crystalline form according to any one of claims 1 to 7.

9. The active pharmaceutical ingredient according to claim 8, **characterized in that** the weight percentage of the crystalline form in the active pharmaceutical ingredient is from 80.0% to 100%.

10. A composition **characterized in that** it comprises the crystalline form according to any one of claims 1 to 7, and one or more pharmaceutically acceptable excipients.

11. The composition according to claim 10, **characterized in that** the composition is a sterile powder, and/or, the pharmaceutically acceptable excipient is a buffering agent.

12. Use of the crystalline form according to any one of claims 1 to 7, the active pharmaceutical ingredient according to claim 8 or 9, or the composition according to claim 10 or 11 in the preparation of an extracellular histone inhibitor.

13. Use of the crystalline form according to any one of claims 1 to 7, the active pharmaceutical ingredient according to claim 8 or 9, or the composition according to claim 10 or 11 in the preparation of a medicament for treating or preventing a disease selected from the group consisting of: severe pneumonia, sepsis, systemic inflammatory response syndrome, acute pancreatitis, acute kidney injury, acute lung injury, acute liver injury, acute respiratory distress syndrome, ischemia-reperfusion injury, atherosclerosis, deep vein thrombosis, ischemic injury, ischemic stroke, multiple sclerosis, systemic lupus erythematosus, ankylosing spondylitis, psoriatic arthritis, ulcerative colitis, Crohn's disease and rheumatoid arthritis.

14. A method for purifying the compound, sodium β-O-methyl-D-cellobioside heptasulfate, **characterized in that** any of the following methods is used:
(1) Anti-solvent addition purification method, specifically: a first solvent is a good solvent and a second solvent is an anti-solvent, the crude product of the compound, sodium β-O-methyl-D-cellobioside heptasulfate, is purified by using the anti-solvent addition method, to afford a purified compound;
(2) Cooling crystallization purification method, specifically: the compound, sodium β-O-methyl-D-cellobioside heptasulfate, is added into a mixed solvent Y and dissolved at high temperature, followed by cooling crystallization to afford a purified compound; the mixed solvent Y is a mixture of the first solvent and the second solvent;
(3) Slurrying purification method, specifically: the crude product of the compound, sodium β-O-methyl-D-cellobioside heptasulfate, is purified by slurrying purification using a mixed solvent X as the solvent, to afford a purified compound, the mixed solvent X is a mixture of the first solvent and the second solvent;
wherein the first solvent is water, and the second solvent is selected from one or more of C1 to C6 alcohol, acetone and acetonitrile.

15. The method for purifying according to claim 14, **characterized in that** the purified compound comprises the crystalline form A according to any one of claims 1 to 7.

16. The method for purifying according to claim 14, **characterized in that** the C1 to C6 alcohol is selected from one or more of methanol, ethanol, and isopropanol.

17. A method for preparing the crystalline form C according to any one of claims 1 to 7, **characterized by** comprising the steps of adding the compound, sodium β-O-methyl-D-cellobioside heptasulfate, to a solvent Z and stirring, isolating the solids to obtain the crystalline form C; wherein the solvent Z is a mixture of a first solvent and a second solvent; and the first solvent is water and the second solvent is 1,4-dioxane.
